**Europäisches Patentamt**

**European Patent Office**

**Office européen des brevets**

(19)

(11) Veröffentlichungsnummer: **0 110 255 B1**

(12) **EUROPÄISCHE PATENTSCHRIFT**

(45) Veröffentlichungstag der Patentschrift:
**12.06.85**

(21) Anmeldenummer: **83111503.5**

(22) Anmeldetag: **17.11.83**

(51) Int. Cl.⁴: **C 07 C 1/20,** C 07 C 11/02 //
B01J29/04

(54) Verfahren zur Herstellung von C2- bis C4-Olefinen aus Methanol/Dimethylether.

(30) Priorität: **25.11.82 DE 3243520**

(43) Veröffentlichungstag der Anmeldung:
**13.06.84 Patentblatt 84/24**

(45) Bekanntmachung des Hinweises auf die Patenterteilung:
**12.06.85 Patentblatt 85/24**

(84) Benannte Vertragsstaaten:
**BE DE FR GB NL**

(56) Entgegenhaltungen:
**EP - A - 0 011 900
EP - A - 0 072 920
US - A - 4 292 458**

(73) Patentinhaber: **BASF Aktiengesellschaft,
Carl-Bosch-Strasse 38, D-6700 Ludwigshafen (DE)**

(72) Erfinder: **Hoelderich, Wolfgang, Dr., Mannheimer
Strasse 18c, D-6710 Frankenthal (DE)**
Erfinder: **Mross, Wolf Dieter, Dr.,
Anselm-Feuerbach-Strasse 21, D-6711 Frankenthal (DE)**
Erfinder: **Schwarzmann, Matthias, Dr.,
Carl-Bosch-Strasse 54, D-6703 Limburgerhof (DE)**

## Beschreibung

In neuerer Zeit gewinnen Bemühungen, Methanol zur Herstellung von Olefinen zu verwenden, zunehmendes Interesse. Methanol lässt sich aus Kohle, über Kohlevergasung und Herstellung von Synthesegas, mit Hilfe bewährter Technologien leicht herstellen. Gelingt es, Methanol in wirtschaftlicher Weise in niedere Olefine umzuwandeln, dann können die heute üblichen Weiterverarbeitungsverfahren der chemischen Industrie auch bei der Verwendung von Kohle als Rohstoff beibehalten werden. In den vergangenen Jahren sind daher Verfahren entwickelt worden, die die Herstellung von Olefinen aus Methanol und/oder Dimethylether zum Gegenstand haben.

Ein solches Verfahren ist beispielsweise in der DE-OS Nr. 2615150 beschrieben. Als Katalysator wird ein Aluminosilikatzeolith ZSM-5 verwendet, der eigentlich ein Aromatisierungskatalysator ist. Die Umsetzung kann aber durch verschiedene Massnahmen, insbesondere durch die Verkürzung der Verweilzeit, in Richtung Olefinbildung gelenkt werden. Weitere, die Olefinbildung begünstigende Parameter sind insbesondere die Verdünnung von Methanol bzw. Dimethylether mit Inertgasen bzw. Wasserdampf oder die Verdünnung des Katalysators mit Bindemitteln. Die Erfahrung zeigt, dass hohe Olefinausbeuten nur durch eine sehr starke Verdünnung von Methanol und/oder Dimethylether mit Inertgas oder Wasserdampf zu erzielen sind. Andere bekannte Verfahren haben als Nachteil eine geringe Belastbarkeit und eine schnelle Verkokung des Katalysators. Es besteht grosses Interesse an einem einfachen Verfahren, das die vollständige Umsetzung von Rohmethanol und/oder Dimethylether in überwiegend aus $C_2$- bis $C_4$-Olefinen bestehende Kohlenwasserstoffe ermöglicht.

Es wurde nun gefunden, dass man $C_2$- bis $C_4$-Olefine in hoher Ausbeute aus Methanol und/oder Dimethylether durch katalytische Umsetzung bei erhöhter Temperatur in Gegenwart von Zeolithkatalysatoren erhält, wenn man als Katalysatoren Borosilikatzeolithe verwendet, die mit Fluorwasserstoff behandelt und danach mit amorphen Aluminosilikaten als Bindemittel verformt und anschliessend mit Salzsäure behandelt werden.

Vorteilhaft verwendet man Katalysatoren, bei denen die Borosilikatzeolithe mit Flusssäure, bevorzugt etwa 0,1N behandelt und mit amorphen Aluminosilikaten im Gewichtsverhältnis 60:40 zu Strängen gepresst werden, und danach eine Salzsäurebehandlung, vorzugsweise mit etwa 18%iger Salzsäure, erfolgt. Erfindungswesentlich ist, dass die Salzsäurebehandlung nach der Verformung der Zeolithe mit dem Bindemittel durchgeführt wird.

Als Bindemittel kann man vorteilhaft amorphe Aluminosilikate, deren $SiO_2/Al_2O_3$-Verhältnis im Bereich von 35:65 bis 75:25 Gew.-% liegt, verwenden.

Ein bevorzugtes Verfahren zur Herstellung der erfindungsgemäss verwendeten Katalysatoren besteht darin, die Zeolithe nach der Calcination 1 bis 3 h zwischen 60 und 80°C einer 0,001 bis, bevorzugt 0,05 bis 0,2N HF, zu behandeln. Nach Abfiltrieren und Auswaschen wird dieses Produkt bei 100 bis 140°C getrocknet und bei 500 bis 600°C calciniert. Der so behandelte Borosilikatzeolith wird mit amorphen Aluminosilikaten, deren $SiO_2/Al_2O_3$-Verhältnis im Bereich von 35:65 bis 75:25, vorzugsweise 75:25 Gew.-%, liegt, im Gewichtsverhältnis 60:40 zu Strängen gepresst und bei 500 bis 600°C erneut calciniert. Nach der Verstrangung mit Bindemittel wird der Katalysator noch 1 bis 3 h zwischen 60 und 80°C in einer 3 bis 25, insbesondere mit einer 12 bis 20%igen Salzsäure behandelt und anschliessend bei 500 bis 600°C 5 h calciniert.

Bei der Durchführung des Verfahrens wird Methanol und/oder Dimethylether bei einem Druck zwischen Normaldruck und etwa 30, vorzugsweise bei 0 bis 1 bar, und bei Temperaturen zwischen 300 und 650, vorzugsweise bei 400 bis 550°C, an den oben beschriebenen Katalysatoren umgesetzt. Das Methanol kann einen Wassergehalt bis zu 90 Gew.-% haben; zweckmässig verwendet man als Ausgangsstoff Rohmethanol, das etwa 20% Wasser enthält.

Dem Methanol können auch noch andere niedere Alkohole beigemischt sein. Die Belastung des Katalysators, ausgedrückt in WHSV= $h^{-1}$ — Gramm Methanol und/oder Dimethylether pro Gramm Katalysator und Stunde —, wird zweckmässig so gewählt, dass die Ausgangsstoffe möglichst quantitativ umgesetzt werden, so dass keine Abtrenn- und Rückführprobleme für nicht umgesetzten Dimethylether entstehen. Im allgemeinen soll daher die WHSV im Bereich von 0,5 bis 50, vorzugsweise im Bereich von 2 bis 15 $h^{-1}$ liegen.

Durch das erfindungsgemässe Verfahren wird die Olefinselektivität im $C_2$- bis $C_4$-Bereich bei der Methanolumwandlung zu Kohlenwasserstoffen, insbesondere im Temperaturbereich von 400 bis 600°C, wesentlich erhöht.

Ein weiterer Vorteil des erfindungsgemässen Verfahrens ist die Erhöhung der Laufzeit der verwendeten Katalysatoren. Unter Laufzeit versteht man die Zeit zwischen zwei Regenierungen. Auch die Lebensdauer des Katalysators wird insgesamt verlängert. Der erfindungsgemässe Effekt der Laufzeitverbesserung wirkt sich besonders bei der Umsetzung bei hohen Temperaturen, z.B. im Bereich von 450 bis 550°C, aus.

Die Bildung der unerwünschten Nebenprodukte Methan und Aromaten wird stark zurückgedrängt. Es ist ein weiterer Vorteil der Erfindung, dass man die Umsetzung zu $C_2$- bis $C_4$-Olefinen mit Rohmethanol ohne weiteren Zusatz von inertem Verdünnungsmittel wie $N_2$, He oder $H_2O$ ausführen kann.

Die Durchführung des erfindungsgemässen Verfahrens wird anhand des nachstehenden Beispiels näher erläutert.

*Beispiel*
*Katalysator A (verwendet beim erfindungsgemässen Verfahren)*

Der Borzeolith wird in einer hydrothermalen Synthese aus 64 g $SiO_2$ (Aerosil 200), 12,2 g $H_3BO_3$, 800 g einer wässerigen 1,6-Hexandiaminlösung (Mischung 50:50) bei 170°C unter autogenem Druck in einem Rührautoklaven hergestellt. Nach Abfiltrieren und Auswaschen wird das kristalline Reaktionsprodukt bei 110°C/24 h getrocknet und bei 500°C/24 h calciniert. Dieser Borosilikatzeolith setzt sich zusammen aus 94,2 Gew.-% $SiO_2$ und 2,32 Gew.-% $B_2O_3$.

50 g dieses Borosilikatzeolithen werden mit 140 ml 0,1N HF unter Rückfluss 1 h behandelt. Nach der Filtration und Auswaschen mit Wasser wird bei 110°C/16 h getrocknet und bei 500°C/ 5 h calciniert. Dieses Produkt wird mit amorphem Aluminosilikat (75% $SiO_2$:25% $Al_2O_3$) im Gewichtsverhältnis 60:40 verstrangt, bei 110°C/16 h getrocknet und bei 500°C/16 h calciniert.

50 g dieser Katalysatorstränge werden mit 200 ml 18%iger HCl 1 h unter Rückfluss behandelt. Der Katalysator wird dann chloridfrei gewaschen, bei 110°C/16 h getrocknet und bei 500°C/5 h geglüht.

An diesem Katalysator A wird unter isothermen Bedingungen in einem Rohrreaktor Rohmethanol mit 20 Gew.-% Wasser bei 500°C und WHSV= 7,8 $h^{-1}$ bzw. auf eingesetztes $CH_3OH$ quantitativ umgesetzt. Die Ausbeuten, bezogen auf eingesetztes $CH_2$, sind in der Tabelle, Spalte A, angegeben.

Zum Ausbeutevergleich wurden die folgenden Katalysatoren herangezogen, die unter denselben Reaktionsbedingungen wie Katalysator A getestet wurden.

*Katalysator B* wird erhalten durch Verstrangen des oben beschriebenen Borosilikatzeolithen mit Böhmit im Gewichtsverhältnis 60:40. Die Trocknung erfolgt bei 110°C/16 h und die Calcinierung bei 500°C/16 h.

*Katalysator C* wird erhalten durch einstündige Behandlung von 70 g Katalysator B mit 465 ml 18%iger Salzsäure unter Rückfluss, danach mit Wasser chloridfrei gewaschen, bei 110°C/16 h getrocknet und bei 500°C/5 h calciniert. Dieser Katalysator entspricht dem Katalysator A ohne HF-Behandlung und ohne Verstrangung mit amorphem Aluminosilikat.

*Katalysator D* wird hergestellt durch Verstrangung von 72 g des beschriebenen Borosilikatzeolithen mit 48 g eines amorphen Aluminosilikates, das sich aus 75% $SiO_2$ und 25% $Al_2O_3$ zusammensetzt. Die 2-mm-Stränge werden bei 110°C/16 h calciniert. 42 g dieser Stränge werden mit 200 ml einer 18%igen Salzsäure unter Rückfluss 1 h erhitzt, danach chloridfrei gewaschen, bei 110°C getrocknet und bei 500°C/5 h getempert.

Dieser Katalysator entspricht dem Katalysator A ohne HF- und HCl-Behandlung.

*Katalysator E* entspricht dem Katalysator A ohne HCl-Behandlung.

Aus dem Vergleich in der Tabelle geht hervor, dass der erfindungsgemässe Katalysator A die besten Ausbeuten an $C_2$ bis $C_4$-Olefinen liefert.

*Tabelle*

| Zusammensetzung der Produkte | Katalysator A | Vergleichskatalysatoren | | | |
|---|---|---|---|---|---|
| | | B | C | D | E |
| $CH_4$ % | 0,9 | 5,4 | 2,5 | 1,1 | 1,5 |
| $C_2H_4$ % | 5,3 | 7,0 | 7,3 | 4,9 | 7,4 |
| $C_2H_6$ % | — | 0,5 | 0,2 | 0,1 | 0,2 |
| $C_3H_6$ % | 47,1 | 31,4 | 41,8 | 41,9 | 42,2 |
| $C_3H_8$ % | 0,6 | 1,6 | 1,0 | 0,6 | 1,1 |
| $C_4H_8$ % | 26,6 | 17,6 | 21,8 | 22,7 | 24,1 |
| $C_4H_{10}$ % | 1,3 | 1,8 | 1,0 | 1,2 | 1,7 |
| $C_5^+$-Aliphaten | 16,2 | 18,9 | 17,9 | 20,8 | 15,2 |
| $C_6^+$-Aromaten | 1,0 | 13,8 | 5,4 | 4,7 | 1,4 |
| Laufzeit (h) | 132 | 12 | 77 | 25 | 53 |
| g $CH_3OH$/g Katalysator | 1030 | 94 | 600 | 195 | 413 |

**Patentansprüche**

1. Verfahren zur Herstellung von $C_2$ bis $C_4$-Olefinen durch katalytische Umsetzung von Methanol und/oder Dimethylether in Gegenwart von Zeolithkatalysatoren bei erhöhter Temperatur, dadurch gekennzeichnet, dass man als Katalysatoren Borosilikatzeolithe verwendet, die mit Fluorwasserstoff behandelt, danach mit amorphen Aluminosilikaten als Bindemittel verformt und anschliessend mit Salzsäure behandelt werden.

2. Verfahren nach Anspruch 1, dadurch gekennzeichnet, dass man die Flusssäurebehandlung der Borosilikatzeolithe mit 0,001 bis 1N HF durchführt.

3. Verfahren nach den Ansprüchen 1 und 2, dadurch gekennzeichnet, dass man als Bindemittel für die Verformung ein amorphes Aluminosilikat verwendet, dessen $SiO_2/Al_2O_3$-Verhältnis im Bereich von 35:65 bis 75:25 liegt.

4. Verfahren nach den Ansprüchen 1 bis 3, dadurch gekennzeichnet, dass man bei der abschliessenden Behandlung der Katalysatoren Salzsäure im Konzentrationsbereich von 2 bis 25% einsetzt.

## Claims

1. A process for the preparation of $C_2$-$C_4$-olefins by catalytic conversion of methanol and/or dimethyl ether in the presence of a zeolite catalyst at elevated temperature, wherein the catalyst used is a borosilicate zeolite which is treated with hydrogen fluoride, molded together with an amorphous aluminosilicate as binder and then treated with hydrochloric acid.

2. A process as claimed in Claim 1, wherein 0.001 to 1N HF is used in the treatment of the borosilicate zeolite.

3. A process as claimed in Claims 1 and 2, wherein the binder used for molding is an amorphous aluminosilicate whose $SiO_2/Al_2O_3$ ratio is from 35:65 to 75:25.

4. A process as claimed in Claims 1 to 3, wherein from 3 to 25% strength hydrochloric acid is used in the final treatment of the catalyst.

## Revendications

1. Procédé pour la préparation d'oléfines en $C_2$-$C_4$ par transformation catalytique de méthanol et/ou d'oxyde diméthylique en présence de catalyseurs zéolitiques à température élevée, caractérisé en ce qu'on utilise, comme catalyseurs, des zéolites de borosilicate qui sont traitées par l'acide fluorhydrique, puis façonnées avec des aluminosilicates amorphes servant de liant et ensuite traitées par l'acide chlorhydrique.

2. Procédé selon la revendication 1, caractérisé en ce qu'on effectue le traitement par l'acide fluorhydrique des zéolites de borosilicate avec un HF 0,001 à 1N.

3. Procédé selon les revendications 1 et 2, caractérisé en ce qu'on utilise, comme liant pour le façonnage, un aluminosilicate amorphe dont le rapport $SiO_2/Al_2O_3$ se situe dans la gamme de 35:65 à 75:25.

4. Procédé selon l'une des revendications 1 à 3, caractérisé en ce que, pour le traitement final des catalyseurs, on utilise un acide chlorhydrique dont la concentration se situe dans la gamme de 3 à 25%.